# EUROPEAN PATENT SPECIFICATION

(11) **EP 1 562 617 B1**
(45) Date of publication and mention of the grant of the patent: **20.12.2006**
(21) Application number: 02779827.1
(22) Date of filing: 06.11.2002
(51) Int. Cl.: A61K 31/36, A61K 36/67, A61K 131/00, A61P 3/06, A61P 3/10

(54) **NEW USE OF PIPATALINE**
NEUE VERWENDUNG VON PIPATALIN
NOUVELLE UTILISATION DE PIPATALINE

(43) Date of publication of application: 17.08.2005
(73) Proprietor: COUNCIL OF SCIENTIFIC AND INDUSTRIAL RESEARCH, New Delhi 110 001 (IN)
(72) Inventor: RAO, Janaswamy, Madhusudana, Hyderabad 500 007, Andhra Pradesh (IN); SRINIVAS, Pullela, Venkata, Hyderabad 500 007, Andhra Pradesh (IN); ANURADHA, Vummenthala, Hyderabad 500 007, Andhra Pradesh (IN); TIWARI, Ashok, Kumar, Hyderabad 500 007, Andhra Pradesh (IN); ALI, Amtul, Zehra, Hyderabad 500 007, Andhra Pradesh (IN); YADAV, Jhillu, Singh, Hyderabad 500 007, Andhra Pradesh (IN); RAGHAVAN, Kondapura, Vijaya, Hyderabad 500 007, Andhra Pradesh (IN)
(74) Representative: Goulard, Sophie
(86) International application number: PCT/IB2002/004654
(87) International publication number: WO 2004/041295

(56) References cited:
- DE-A- 3 724 341
- GB-A- 2 314 270
- DATABASE WPI Section Ch, Week 199313 Derwent Publications Ltd., London, GB; Class B02, AN 1993-104193 XP002228532 & JP 05 043458 A (SUNTORY LTD), 23 February 1993 (1993-02-23)
- DATABASE WPI Section Ch, Week 200167 Derwent Publications Ltd., London, GB; Class B05, AN 2001-592416 XP002228533 & JP 2001 151676 A (KUKI SANGYO KK), 5 June 2001 (2001-06-05)
- STÖHR, J.R.; XIAO, P.-G.; BAUER, R.: "Constituents of Chinese Piper species and their inhibitory activity on prostaglandin and leukotriene biosynthesis in vitro." JOURNAL OF ETHNOPHARMACOLOGY, vol. 75, no. 2-3, 2001, pages 133-139, XP001122249
- VIRINDER S. PARMAR ET AL.: "Phytochemistry of the genus Piper" PHYTOCHEMISTRY, vol. 46, no. 4, 1997, pages 597-673, XP004293460
- ASHOK PUROHIT AND H.M.M. DARADKA: "Anti-diabetic efficacy of Piper longum fruit (50% EtOH extract) on Alloxan induced diabetic rats." JOURNAL OF THE DIABETIC ASSOCIATION OF INDIA, vol. 38, no. 1, 1999, pages 22-23, XP001135013

## Description

This invention describes a method for providing α-glucosidase inhibition to a subject by administering a pharmaceutical composition comprising pipataline (formula 1a). In particular this invention relates to the isolation of pipataline [5-(1-dodecenyl)-1, 3-Benzodioxol] from the plant source *Piper longum* in significant yields. This invention also identifies the therapeutic application of this compound as α-glucosidase inhibitors in the form of suitable pharmaceutical composition for the treatment of diseases like, hyperlipoproteinemea, viral infection, hepatitis B and C and HIV.

### Background Art

The use of plants as medicines goes back to early man. Certainly the great civilization of the ancient Indians, Chinese and North Africans provided written evidences of man's ingenuity in utilizing plants for the treatment of a wide variety of diseases (Phillipson J. D *phytochemistry,* 2001, 56, 237-243). As, new research and clinical experience is broadening the knowledge, changes in drug therapy are also being observed. Due to the occurrence of new diseases and identification of targets with multiple therapeutic applications there is an ongoing search for new compounds having unique structures and properties.

α-glucosidase enzyme has been identified as such a target. Inhibitors of a-glucosidase are increasingly finding therapeutic application in metabolic disorders such as diabetes mellitus, obesity, hyperlipoproteinemia Type IV (Trusch eit, E. *et al., Angew. Chem.. Int. Ed.* Engl. 1981,20, 744-761; Puls, W. Keupu *Diabelologia,* 1973, 9, 97; Puls, W Habilitationssoh Chrift universitat Dusseldorf 1980), HIV, human hepatitis B virus, human cytomegalovirus and influenza (Heightman T D and vasella A T, *Angew. Chem. Int. Ed. Engl.* 1999, 38, 750- 770; Mehta *et al., FEBS Lett.* 1998,430, 17-22; Watson A. A *et al., Phytochemistry* 2001, 56, 265-295), cancer and in immuno compromised cases. The serum level of glucosidases has been found to be increased in many patients with different tumors (Woollen, J. W and Tesiar, ' P. 1965, *'din. Chem. Ada.* 12, 671-683) and are being realized to be involved in the degradation of the extracellular matrix, in tumor cell invasion (Bemaki, R. J *et al.,* 1985 Cancer metastasis Rev 4, 81-102). Therefore, inhibitors of catabolic glucosidases are being actively pursued as a therapeutic strategy for cancer (Watson, A. A *et al., Phytochemistry* j 2001, 56, 265-295).

Variety of compounds bearing a-glucosidase inhibiting potential has been reviewed for their chemotherapeutic values (El Ashry *et al., Pharmazie,* 2000, 55, 251-262, 331-348 and 403-415). Although several drugs targeted for a-glucosidase inhibition are either in clinical practice or various stages of clinical development (Drugs of the future 1986, 11, 795-797; Dugs of the future 1986, 11, 1039-1042; Watson A. A *et al., Phytochemistry* 2001, 56, 265-295) the impact of the burden of diseases as discussed above underscores the clear need for new agents. It is also necessary to have a large inhibitors pool as patients can develop resistance to current regimens.

Historically, the knowledge gained from traditional medicinal practice and the screening of the extracts from the plants and as animals there in has yielded novel natural products which themselves as potential bioactive agents for the treatment of human diseases (Gullo. V.P, The discovery of natural products with therapeutic potential, Butterworth-Heinemann, Boston, 1994; Cragg G. Metal J. Nat. Prod. 1997, 60: 52-60).

The screening of natural sources has led to the discovery of many clinically useful drugs that play a major role not only in the treatment of diseases discussed above, but also in the prevention of such diseases. Therefore, increasing clinical importance of epidemics of diabetes, cancer, HIV and other viral diseases as well as drug resistance has led additional urgency to identify novel resources for active compounds to have a large pool.

As described hereafter, our search for a-glucosidase inhibitors from traditional medicinal plants has led to the identification *of Piper longum* which contained in significant yield potent a-glucosidase inhibitors.

*Piper longum* Linn. (Pippali) has been described in traditional medical practice of India for malarial fever, heart disease, splenomegaly, cough, oeadema and so on (P.V. Sharma, Classical uses of medicinal plants, Haridas Ayurveda series (4), Chaukambha Viswabharathi, Varanasi, 1996).

The present invention relates to the identification and isolation of pipataline (Ia) as a potent a-glucosidase inhibitor from *Piper longum* in the form of suitable pharmaceutical composition which may find therapeutic application in the treatment of diseases like, hyperlipoproteinemea, viral infection, hepatitis B and C and HIV.

Various piper species from which compound claimed in this invention as α-glucosidase inhibitors have been tabulated in table 1 and their biological activities in table 2.

### Application and administration:

The α-glucosidase inhibitor of this invention can be applied or administered by any conventional method to the management and treatment of, HIV, AIDS, Hepatitis B and or C, other viral infections.

For human, animals and/or veterinary application compounds as a-glucosidase inhibitor of the invention may be administered through various routes as per the suitability and clinical condition. For human application compounds as α-glucosidase inhibitor may be administered through various routes including oral, intraperitoneal, intravenous, and/or intramuscular as per the case may be.

### Formulations:

The compound as a-glucosidase inhibitor of this invention may be formulated with any of the pharmaceutically applicable additive, carrier vehicle that by no means should alter the potency and property of the compound in any form.

For human applications, the compound of this invention as α-glucosidase inhibitor may be formulated with many of the pharmaceutically acceptable carriers and additives useful for administration of pharmaceutical, which is well known in the art.

The selected carriers or vehicles would of course be consistent with the mode of application or administration of glucosidase inhibitors.

### Effective levels:

The expressions "an effective amount" and or "a suppressive amount" are used to that quantity of the a-glucosidase inhibitor compound of the invention which appears necessary to obtain a reduction in the level of disease, such as suppression of viral infection significantly, relative to that occurring in an untreated control under suitable conditions of the treatment as per the disease condition and severity. It implies that an effective amount of the a-glucosidase inhibitor compound of this invention would be less than any amount that would induce significant unwanted side effects in the organism being treated for a particular disease. This implication is reinforced by the use of the expression "pharmaceutically effective amount". The actual rate and amount of application may vary depending on the disease conditions. This may be irrespective of the concentrations as described in the examples of the invention.

The actual rate and amount of application may vary depending upon the disease and /or infection severity and may also depend upon the plurality of the factors like age and sex of the individual being treated and the mode of administration etc. Upon taking these factors into account, actual dose level and regimen could be readily determined by the person of ordinary skill in the art.

**Table 1:**

| S.No | COMPOUND NAME | PLANT SOURCE | REFERENCE |
|---|---|---|---|
| 1. | Pipataline | *Piper brachystachyum* | *Phytochemistry,* 1988, 27, 3523. |
| | | *Piper peepuloides* | *Planta Medico,* 1973, 23, 295. |
| | | *Piper sylvaticum* | *Phytochemistry,* 1990, 29, 2733 |
| 2. | Sesamin | *Piper brachystachyum* | *Indian Journal of Chemistry,* 1976, 14B, 389. |
| | | *Piper guineense* | *Journal of the Chemical Society, Perkin transactions I,* 1974, 19, 2195. |
| | | *Piper longum* | *Indian Journal of Chemistry,* 1966, 4, 252. |
| | | *Piper lowong* | *Phytochemistry,* 1993, 33, 523. |
| | | *Piper peepuloides* | *Planta Medica,\913,* 23, 295. |
| | | *Piper sylvaticum* | *Phytochemistry,* 1974, 13, 2327 |
| | | *Piper retrofractum* | *Phytochemistry,* 1 985,24,279 |
| 3. | Pellitorine | *Piper attenautunt* | *Indian Journal of Chemistry* 1979, 17B, 538. |
| | | *Anacyclus pyrethrum* | *J. Am. Chem. Soc.,1949,* 71, 366-7. |
| | | *Piper chaba* | *Fltoterapia,* 1995, 66, 188. |
| | | *Piper guineense* | *Toxicon,* 1992.30, 1037. |
| | | *Piper longum, Piper peepuloides* | *Indian Journal of Chemistry,* 1967, 5, 588. |
| | | *Piper nepalens* | *Phytochemistry,* 1972, 11, 2646. |
| | | *Piper nigrum* | *Journal of Agricultural and Food Chemistry,* 1981, 29, 115. |
| | | *Piper ribesioides* | *Plata Medica,* 1989, 55,193. |
| | | *Piper sarmentosum* | *Tetrahedron,* 1987, 43, 3689. |
| | | *Piper sylvaticum* | *Experientia,* 1974, 30, 223. |
| | | *Fagara xanthoxylodea* | *Journal of Chemical Society,* 1963, 3503-5. |
| 4. | Guineensine | *Piper attenauium* | *Indian Journal of Chemistry* 1979, 17B, 538. |
| | | *Piperguineense* | *Journal of the Chemical Society, Perkin transactions I,* 1974, 19, 2195. |
| | | *Piper brachys tachyum* | *Phytochemistry,* 1988, 27, 3523. |
| | | *Piper longum* | *Chem.pharnta.BulL,* 1983, 31, 3562. |
| | | *p. nigrum* | *Chem.pharma.BulL,* 1988,36,2452 |
| | | *Piper officinarum* | *Phytochemistry,* 1976,15,425. |
| | | *Piper sylvaticum* | *Indian Journal of Chemistry* 1980, 19B, 346. |
| 5. | Brachyslamide-B | *Piper brachystachyum* | *Phytochemistry,* 1989, 28, 3039. |
| | | *Piper longum* | *Nat.Prod.Sci,,* 4(1), 23-25, 1999. |

Piper compounds show a wide range of biological activities. Biological activities of pipataline, sesamin, pellilorine, guineensine and brachystamide-B are depicted in Table 2.

**Table 2:**

| S.No. | COMPOUND NAME | BIOLOGICAL ACTIVITY | REFERENCE |
|---|---|---|---|
| 1. | Pipataline | - | - |
| 2 | Sesamin | Anti oxidant | *Spec-pub! ~R- Sac. Chew.,* 181, 230-5,19§6 |
| | | Anti fungal | *J. Chem. Ecol.,* **22**(7), 1325-1330, 1998. |
| | | Anti bacterial | *Fitoterapia JQ (\),* 89-92, 1999. |
| | | Live protective, antioxidant | *Food Style.,* 21**,**2(12), 35-38. |
| | | Anti feedant | *Fitoterapia,* 72 (5), 538-543. |
| | | Inhibition of breast cancer | *JP 5043458 A (Abstract)* |
| | | Apoptosis inducer | *JP2001151676 A (Abstract)* |
| 3. | Pellitorine | Insecticidal activities against Moth fly (Telmatoscopus Albipunctatus). | *Pestc.sci.1991,* 18(3), 21 1-21. |
| | - | Insecticidal against Musca Domestica. | *Toxicon,* 1992.30, 1037. |
| | | Insectgrowth inhibitor against p. gossipiella, H. virescens, H. zea | *Experientia* 1984, 40(4), 340-1. |
| | | Larvicida activity against Aedus Eriseratus larvae. | *J. Chem. Ecol.* 1980, 6(1), 35-48. |
| | | Antituberculotic against 8 Mycobacterium stains | *Bull.Med Ethno Bot.Res.* 1980, (1), 99-106. |
| | | Ovicidal against Leplinotarsa dectmlineata | *Biosci.Biotechnol.Biochem.,* 1994,58(5), 936-7 |
| | | Local anaesthetic | *Journal of Chemical Society** 1963,3503-5- |
| | | Insecticidal against Collosobruchus chinenses | *Journal of Agricultural and Food Chemistry,* 1981, 29, 1 15. |
| | | Antifungal against Cladosporiumsphaerospermum | *Phytochemistry,* 55(6), 621-626, 2000. |
| 4. | Guineensine | Insectcidal against Collosobruchus chinenses | *Journal of Agricultural and Food Chemistry,* 1981, 29, 1 15. |
| | | Larvicidal against Toxocara canis | *Chem. Pharma. Bull.,* 1988, 36, 2452 |
| | | Insecticidal activity | *J. Ind Chem. Soc.,* 76(11-12), 713-717. |
| 5. | Brachystamide-B | | |

The main object of the invention is to provide a new activity for pipataline obtained from *piper longum* as α-glucosidase inhibitor.

Accordingly, this invention relates to the use of an effective amount of pipataline of formula 1a: 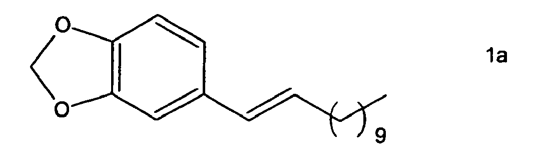 isolated from *Piper Longum,* and of a pharmaceutically acceptable ingredient for the manufacture of a pharmaceutical composition for the management and the treatment of diseases like, hyperlipoproteinemea, viral infection, hepatitis B and C and HIV .

The present invention relates to a new activity for pipataline obtained from *piper longum* as a α-glucosidase inhibitor in the management and treatment of human diseases like hyperlipoproteinemea, viral infection, hepatitis B and C and HIV.

The invention also relates to isolation of pipataline from a new source namely *piper longum.*

In an embodiment of the invention, pipataline provides α-glucosidase inhibitory activity up to 77.45% and has an IC₅₀ value of 26.52 (µg/ml).

The present invention provides a process for isolation of pipataline from *piper longum* for the first time.

The process of isolation of pipataline (formula 1a), comprises the steps of:
a. extraction the dried fruits *of Piper longum* with a solvent,
b. concentrating the extract under vacuum to obtain a residue and
c. eluting the residue of step (b) with hexane to obtain pipataline and a residue.

In an embodiment, the solvent used in step (a) is selected from hexane, cyclohexane or n-pentane.

According to the invention the isolation of pipataline is made from an entirely new source.

### Brief description of the drawings:

The invention is illustrated by the accompanying drawings wherein:
**Figure 1(a)** represents formula of pipataline [5-(1-dodecenyl)-1, 3-benzodioxol];
**Fig. 2(a)** is a graphical representation depicting the α-glucosidase inhibitory activity of pipataline, sesamin, pellitorine, guineensine and brachystamide-B.

Some of the embodiments of the present invention are represented by the following examples, which should not be construed, as limitations on the inventive scope of this invention.

### Example 1

### Experimental protocol: A process for the isolation of pipataline.

The dried, powdered fruits of *Piper longum* (500g) were loaded on a soxhlet apparatus. The powder was extracted with hexane. The hexane extract was concentrated under vacuum. The dark green colored residue was loaded on silica gel column 60-120 mesh, 3.5-cm dia column loaded to a height of 60 cm.
Initially the column was eluted with hexane to get pipataline. The yield of pipataline is around 6.0g.
The above compound was obtained in 90% purity.
The spectrochemical and physical properties of the above said compound is as under:
Pipataline has the following spectrochemical and physical properties.
**Molecular Formula: C₁₉H₂₈O₂**
**MP:** 38°C.
**IR** (KBr) γₘₐₓ cm⁻¹
2829,1468,1248,1040,980,960.
**'H NMR (200 MHz, CDCl₃) (δ)**
0.95 (3H, t, H-1), 1.20-1.60 (16H, b, H-2-9), 2.20 (2H, q, H-10), 5. 90(2H, s, -OCH₂O), 6.0-6.15 (1H, q, H-11), 6.30 (1H, d, J = 15.5 Hz, H-12), 6.70 (2H, s, H-5', 6'), 6.88 (1H, s, H- 2¹).
**¹³C NMR (50 MHz, CDCl₃)**
14.12 (C-1), 22.71 (C-2), 29.37-29.66 (C-3-8), 31.95 (C-9), 32.95 (C-10), 100.89 (--), 105.46 (C-20, 108.20 (C-5¹), 120.17 (C-6^{f}), 129.27 (C-11), 129.57 (C-1 2), 132.61 (C-1'), 146.68 (C-4'), 148.01 (C-3')
**EI-MS**
M⁺ 288

### Example 2:

### Determination of α-Glucosidase inhibition activity of the compound isolated from P. longum:

The α-glucosidase inhibitory assay was done by the chromogenic method. In brief 10µl of test compound dissolved in DMSO (5mg/ml and subsequent dilutions) was incubated for 5 min. with 5µl of yeast α-glucosidase enzyme prepared in 100mM phosphate buffer (pH 7.00). After 5 minutes of incubation, 50fil of 5mM substrate (p-nitrophenyl-α-D-glucopyranoside prepared in the same buffer) was added. The pre-substrate and 5-min post-substrate addition absorbances were recorded at 405nm spectrophotometrically. The increase in absorbance from pre-substrates addition to post substrates reaction was obtained. Percent inhibition was calculated by (1-O.D test/O.D control)x 100 and inhibitory concentration 50% (IC50) was calculated by applying suitable regression analysis.

In accordance with the practice of this invention, it has been found that pipataline, is isolated from *Piper longum* which is an entirely new source. The yield of this compound is also substantial. Also, it has been found that the above said compound shows a-glucosidase inhibition property.

### Advantages:

α-glucosidase inhibitors recently have attracted the attention due to their broad-spectrum activities in disorders of multiple origin viz., viral disorders, HIV, Hepatitis- B and C etc. Much attention being directed now to procure the α-glucosidase inhibitors from natural sources.

The compound pipataline is used in pure form. Hence, isolation of pipataline from *Piper longum* in significant yields as a-glucosidase inhibitor makes the invention very important.

## Claims

1. Use of an effective amount of pipataline of formula 1a: 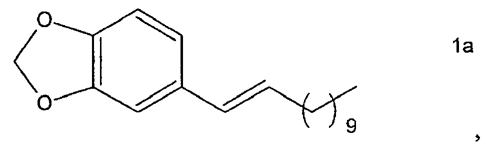 isolated from *Piper longum* and of a pharmaceutically acceptable ingredient for the manufacture of a pharmaceutical composition for the management and the treatment of diseases like, hyperlipoproteinemia, viral infection, hepatitis B and C and HIV.

2. The use of claim 1, wherein pipataline has an α-glucosidase inhibitory activity up to 77.45%.

3. Use as claimed in claim 1 or 2, wherein pipataline has IC₅₀ value of 26.52 µg/ml.

## Patentansprüche

1. Verwendung einer wirksamen Menge Pipatalin der Formel 1a: 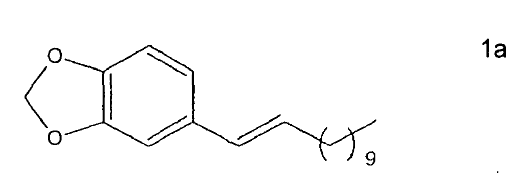 isoliert aus *Piper longum,* und einem pharmazeutisch verträglichen Zusatzstoff zur Herstellung einer pharmazeutischen Zusammensetzung zum Management und zur Behandlung von Erkrankungen wie Hyperlipoproteinämie, Virusinfektion, Hepatitis B und C und HIV.

2. Verwendung nach Anspruch 1, wobei Pipatalin eine α-Glucosidasehemmende Wirkung von bis zu 77,45% hat.

3. Verwendung nach Anspruch 1 oder 2, wobei Pipatalin einen IC₅₀-Wert von 26,52 µg/ml hat.

## Revendications

1. Utilisation d'une quantité efficace de pipataline de formule 1a : 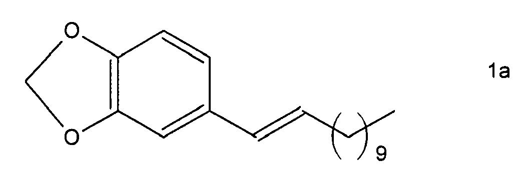 isolée de *Piper longum* et d'un ingrédient pharmaceutiquement acceptable pour la fabrication d'une composition pharmaceutique destinée à la prise en charge et au traitement de maladies telles que l'hyperlipoprotéinémie, une infection virale, l'hépatite B et C et une infection par le VIH.

2. Utilisation selon la revendication 1, dans laquelle la pipataline possède une activité d'inhibition de l'α-glucosidase pouvant atteindre jusqu'à 77,45 %.

3. Utilisation selon la revendication 1 ou 2, dans laquelle la pipataline a une valeur de CI₅₀ de 26,52 µg/mL.
